# FASCICULE DE BREVET EUROPEEN

(11) **EP 3 955 893 B1**
(45) Date de publication et mention de la délivrance du brevet: **07.06.2023**
(21) Numéro de dépôt: 20720393.6
(22) Date de dépôt: 15.04.2020
(51) Int. Cl.: A61K 9/08, A61K 47/10, A61K 47/18, A61K 31/137, A61P 9/04

(54) **SOLUTION PHARMACEUTIQUE D'ADRÉNALINE POUR DISPOSITIF D'INJECTION**
PHARMAZEUTISCHE LÖSUNG VON ADRENALIN FÜR EINE INJEKTIONSVORRICHTUNG
PHARMACEUTICAL SOLUTION OF ADRENALIN FOR INJECTION DEVICE

(30) Priorité: 18.04.2019 FR 1904166
(43) Date de publication de la demande: 23.02.2022
(73) Titulaire: Crossject, 21000 Dijon (FR)
(72) Inventeur: TEILLAUD, Eric, 21121 DAIX (FR); BAUMARD, Nicolas, 21000 DIJON (FR); RESSEJEAC, Audrey, 21850 SAINT APOLLINAIRE (FR)
(74) Mandataire: Germain Maureau
(86) Numéro de dépôt international: PCT/EP2020/060523
(87) Numéro de publication internationale: WO 2020/212381

(56) Documents cités:
- WO-A2-03/041687
- FR-A1- 2 779 061
- FR-A1- 3 014 317

## Description

L'invention concerne une solution pharmaceutique d'adrénaline (aussi connue sous le nom d'épinéphrine) ou d'un sel pharmaceutiquement acceptable de celle-ci (ci-après abrégée « solution pharmaceutique d'adrénaline ») destinée à être injectée par voie parentérale, notamment intramusculaire.

L'adrénaline est une hormone secrétée par le système nerveux central et également par les glandes surrénales. De fortes émotions telles que la peur ou la colère entraînent la libération d'adrénaline dans la circulation sanguine ; ce qui provoque l'augmentation du rythme cardiaque, la tension musculaire, la pression artérielle, ainsi que du métabolisme du sucre.

En médecine, l'injection d'adrénaline ou de sels d'adrénaline est connue pour être un traitement de choix des arrêts cardio-circulatoires, des chocs anaphylactiques (liés à une allergie) ou certains autres états de choc graves.

Par exemple, en cas de choc anaphylactique, le patient peut lui-même s'injecter en intramusculaire de l'adrénaline avec un dispositif d'injection. En effet, l'anaphylaxie est une réaction allergique généralisée soudaine et sévère qui peut être fatale en quelques minutes en l'absence d'un traitement adéquat.

L'adrénaline et ses sels appartiennent à la famille des catécholamines.

L'adrénaline, ainsi que ses sels sont sensibles à l'oxydation. Plus précisément, les solutions aqueuses d'adrénaline se détériorent rapidement lorsqu'elles sont exposées à l'air, la lumière et/ou la chaleur. Cette détérioration est visible du fait qu'elles se décolorent en rose à cause de leur oxydation en adénochrome, puis en brun avec la formation de mélamine.

C'est pourquoi, du fait de cette auto-oxydation de l'adrénaline, il est indispensable de mettre en oeuvre des mesures appropriées qui évitent sa détérioration. Par exemple, il est connu de stocker les solutions d'adrénaline sous réfrigération adéquate afin de prolonger leur durée de conservation.

Il est en outre connu de préserver l'adrénaline de l'auto-oxydation en la combinant avec un antioxydant. De nombreux antioxydants ont été utilisés pour stabiliser des solutions pharmaceutiques d'adrénaline, dont notamment des solutions injectables. Parmi ces antioxydants, on peut citer les sulfites (notamment les métabisulfites), l'acide ascorbique, le thioglycolate, le thioglycérol, la L-cystéine, le propyl gallate, le sulfoxylate de formaldéhyde, l'acide citrique et le monothioglycérol.

Cependant, certains de ces antioxydants tels que les composés à base de sulfite (par exemple le métabisulfite de sodium ou le bisulfite de sodium) peuvent être la cause de sévères réactions allergiques. En outre, le bisulfite de sodium peut réagir avec l'adrénaline, réduisant ainsi tout le potentiel de cette substance active, et ce en produisant un sous-produit qui est l'acide sulfonique d'adrénaline.

La mise au point de solutions pharmaceutiques d'adrénaline de longue conservation est complexe, car il faut absolument veiller à mettre en oeuvre des mesures qui tiennent compte que cette substance active peut facilement s'auto-oxyder en présence de chaleur, d'air et/ou de lumière et qu'elle peut réagir avec certains antioxydants tels que les sulfites réduisant ainsi tout son potentiel de traitement.

Les inventeurs de la présente invention ont cherché à surmonter tous les inconvénients détaillés ci-dessus et ont mis au point une nouvelle solution pharmaceutique d'adrénaline ou d'un sel pharmaceutiquement acceptable de celle-ci destinée à être injectée par voie parentérale, en particulier intramusculaire, dont la stabilité au cours du temps est améliorée par rapport aux solutions d'adrénaline connues de l'état de l'art et qui s'affranchit d'antioxydants de type sulfite.

Les inventeurs de la présente invention ont découvert de manière tout à fait surprenante que l'association de l'antioxydant consistant en le succinate de d-alpha-tocophéryl polyéthylène glycol (ci-après abrégé : « vitamine E TPGS ») avec un agent chélatant dans une solution pharmaceutique d'adrénaline ou d'un sel pharmaceutiquement acceptable de celle-ci permettait d'atteindre parfaitement ces objectifs.

La vitamine E TPGS est formée par estérification du succinate acide de d-alpha-tocophéryle avec le polyéthylène glycol (abrégé ci-après « PEG »).

La présente invention a ainsi pour premier objet une solution pharmaceutique d'adrénaline qui comprend au moins :
- de l'adrénaline ou un sel pharmaceutiquement acceptable de celle-ci ;
- de la vitamine E TPGS ;
- un agent chélatant ;
- un solvant.

L'agent chélatant peut être choisi parmi l'éthylènediaminetétraacétate disodique (aussi connu sous la dénomination de sel disodique de l'acide éthylènediaminetétraacétique dihydraté et ci-après abrégé « EDTA disodique »), l'acide éthylènediaminetétraacétique et l'éthylènediaminetétraacétate de calcium disodique.

De manière tout à fait préférée, l'agent chélatant est l'EDTA disodique.

Les inventeurs ont découvert de manière tout à fait surprenante que l'association de la vitamine E TPGS avec un agent chélatant, de préférence de l'EDTA disodique, confère à la solution d'adrénaline une meilleure stabilité au cours du temps que celle de solutions d'adrénaline de l'état de l'art. En effet, les solutions d'adrénaline connues de l'état de l'art ont des dates de péremption comprises entre 18 et 21 mois. La solution d'adrénaline selon l'invention peut avoir une date de péremption supérieure à 24 mois.

La présente invention réside donc dans la sélection d'une association de vitamine E TPGS et d'un agent chélatant (de préférence l'EDTA disodique) pour stabiliser une solution pharmaceutique d'adrénaline.

Il a en effet été constaté au cours d'expérimentations que les solutions pharmaceutiques selon l'invention demeuraient parfaitement stables au cours du temps, et ce même dans des conditions de stockage sévères (à savoir à une température de 40°C). Plus précisément, dans ces conditions de stockage, à la différence d'autres solutions d'adrénaline comparatives, on a relevé que :
- les solutions d'adrénaline selon l'invention demeuraient limpides et incolores;
- leur pH ne variait quasiment pas, et
- le pourcentage de perte de pureté était très faible.

Les inventeurs ont donc constaté un effet synergique entre la vitamine E TPGS et un agent chélatant (de préférence l'EDTA disodique) qui repose sur les faits suivants :
- la vitamine E TPGS évite la dégradation de l'adrénaline par auto-oxydation, tandis que
- l'agent chélatant limite la diminution du pH de ladite solution ; ce qui évite des réactions chimiques au sein de la solution qui seraient susceptibles également de dégrader l'adrénaline.

Le sel pharmaceutiquement acceptable de l'adrénaline peut être choisi parmi le tartrate d'adrénaline et le chlorhydrate d'adrénaline Il peut être pris seul ou en combinaison de ceux-ci.

De préférence, le sel d'adrénaline est le tartrate d'adrénaline.

Le solvant peut être tout solvant pharmaceutiquement acceptable et qui est compatible avec l'adrénaline et ses sels, ainsi que tout autre composé que comprend ladite solution pharmaceutique selon l'invention. Il peut s'agir d'eau, en particulier d'eau utilisée dans les dispositifs d'injection (autrement dit de l'eau pour préparation injectable). L'eau pour préparation injectable est ultra-pure et exempte de contaminants bactériens.

La solution pharmaceutique peut en outre comprendre au moins un agent tampon de pH. Par exemple, il peut s'agir d'un agent tampon de pH choisi parmi l'acide chlorhydrique et la soude.

Le pH de la solution pharmaceutique est avantageusement compris entre 2,2 et 5, de préférence entre 3 et 3,8.

La solution pharmaceutique peut en outre comprendre au moins un modificateur de tonicité. Par exemple, il peut s'agir du chlorure de sodium.

Ladite solution pharmaceutique peut en outre comprendre au moins un excipient pharmaceutique acceptable.

Dans un mode de réalisation de l'invention, la solution pharmaceutique comprend :
- de l'adrénaline ou un sel pharmaceutiquement acceptable de celle-ci ;
- de la vitamine E TPGS ;
- un agent chélatant, de préférence l'EDTA disodique ;
- optionnellement au moins un agent tampon de pH ;
- optionnellement au moins un modificateur de tonicité ;
- un solvant, de préférence de l'eau pour préparation injectable.

Préférentiellement, la solution pharmaceutique comprend :
- de l'adrénaline ou un sel pharmaceutiquement acceptable de celle-ci ;
- de la vitamine E TPGS ;
- de l'EDTA disodique ;
- optionnellement de la soude ou de l'acide chlorhydrique ;
- optionnellement du chlorure de sodium ;
- un solvant, de préférence de l'eau pour préparation injectable.

Plus préférentiellement, la solution pharmaceutique comprend :
- du tartrate d'adrénaline ;
- de la vitamine E TPGS ;
- de l'EDTA disodique ;
- optionnellement de la soude ou de l'acide chlorhydrique ;
- optionnellement du chlorure de sodium ;
- un solvant, de préférence de l'eau pour préparation injectable.

De manière avantageuse, la concentration en adrénaline dans ladite solution pharmaceutique est comprise entre 0,1 mg/mL et 1 mg/mL.

Dans des modes de réalisation de l'invention, la concentration en adrénaline dans ladite solution est de 0,48 mg/mL ou encore de 0,8 mg/mL.

La solution pharmaceutique selon l'invention peut comprendre en mg pour 1 mL de solution :
- entre 0,1 mg et 1 mg, de préférence entre 0,48 mg et 0,8 mg d'adrénaline ou d'un sel pharmaceutiquement acceptable de celle-ci ;
- entre 0,1 mg et 20 mg, de préférence entre 2,5 mg et 15 mg, de vitamine E TPGS ;
- entre 0,1 mg et 2 mg, de préférence entre 1 mg et 1,5 mg, d'agent chélatant ;
- Q.S.P. 1 mL de solvant, de préférence d'eau pour préparation injectable.

« Q.S.P.» étant l'abréviation pour « quantité suffisante pour » et signifie que le solvant doit être ajouté dans le mélange en une quantité suffisante pour que la somme des volumes des constituants du mélange soit égale à 1 mL.

La solution pharmaceutique selon l'invention peut comprendre en mg pour 1 mL de solution :
- entre 0,1 mg et 1 mg, de préférence entre 0,48 mg et 0,8 mg, d'adrénaline ou d'un sel pharmaceutiquement acceptable de celle-ci ;
- entre 0,1 mg et 20 mg, de préférence entre 2,5 mg et 15 mg, de vitamine E TPGS ;
- entre 0,1 mg et 2 mg, de préférence entre 1 mg et 1,5 mg, d'EDTA disodique ;
- Q.S.P. 1 mL de solvant, de préférence d'eau pour préparation injectable.

Ladite solution peut en outre comprendre jusqu'à 10 mg d'au moins un excipient pharmaceutiquement acceptable.

La présente invention a aussi pour objet un procédé de préparation de la solution pharmaceutique selon l'invention telle que décrite ci-dessus qui comprend au moins les étapes suivantes :
a) on prépare sous agitation un mélange comprenant tous les constituants de ladite solution à l'exception de l'adrénaline ou du sel pharmaceutiquement acceptable de celle-ci de manière à les dissoudre ;
b) on dissout dans un solvant l'adrénaline ou un sel pharmaceutiquement acceptable de celle-ci ;
c) on ajoute au mélange de l'étape a), optionnellement sous agitation, l'adrénaline dissoute ou le sel pharmaceutiquement acceptable de celle-ci dissout.

Au cours du procédé de préparation, les étapes mettant en oeuvre une agitation sont avantageusement réalisées à une vitesse d'agitation comprise entre 200 et 400 tour/minute, plus préférentiellement entre 250 et 300 tour/minute.

Le procédé peut être réalisé sous atmosphère d'azote ou d'argon ou bien le mélange peut être bullé à l'azote ou à l'argon.

L'invention a aussi pour objet la solution pharmaceutique telle que décrite ci-dessus pour son utilisation dans le traitement des chocs anaphylactiques, des arrêts cardiaques, de l'asthme et des détresses cardio-circulatoires (notamment les détresses cardio-circulatoires avec états de choc anaphylactique, hémorragique, traumatique, infectieux ou secondaire à la chirurgie cardiaque). De préférence, il s'agit du traitement des chocs anaphylactiques.

Ladite solution est avantageusement sous une forme adaptée à l'administration par voie parentérale, de préférence par voie intramusculaire.

La présente invention a aussi pour objet un kit d'injection, de préférence un kit d'injection par voie intramusculaire, comportant :
- un dispositif d'injection ;
- la solution pharmaceutique selon l'invention telle que décrite ci-dessus.

De manière avantageuse, le volume d'injection du dispositif d'injection est compris entre 0,3125 ml et 0,625 ml.

Ledit dispositif d'injection peut être à usage unique. Par exemple, il s'agit d'un tube pré-rempli prêt à l'emploi.

Dans un mode de réalisation préféré de l'invention, ledit dispositif est un dispositif d'injection pré-rempli, à usage unique, sans aiguille et automatique grâce à un générateur de gaz dont il est équipé. Il peut s'agir d'un dispositif d'injection sans aiguille à cartouche pyrotechnique. A cet égard, les demandes de brevet FR 2 815 544 A1 et FR 2 807 946 A1 décrivent un exemple de ce dispositif d'injection.

De manière tout à fait avantageuse, le dispositif d'injection est un dispositif commercialisé par la société Crossject sous la dénomination commerciale ZENEO^{®}.

Ainsi, dans un mode de réalisation du kit d'injection selon l'invention, le dispositif d'injection est un dispositif d'injection sans aiguille à cartouche pyrotechnique.

### PARTIE EXPERIMENTALE

Des expérimentations ont été réalisées afin de comparer la stabilité de solutions pharmaceutiques d'adrénaline selon l'invention avec celle de solutions pharmaceutiques comparatives qui étaient dépourvues d'antioxydant ou qui comprenaient des antioxydants connus de l'état de l'art pour stabiliser les solutions pharmaceutiques d'adrénaline, éventuellement avec également un agent chélatant qui était de l'EDTA disodique.

Plus précisément, les 19 solutions d'adrénaline suivantes ont été préparées :
- solutions S1 et S2: 2 solutions d'adrénaline selon l'invention ;
- solutions C1 et C2 : 2 solutions d'adrénaline comparatives qui étaient dépourvues de tout antioxydant ;
- solutions C3 et C4 : 2 solutions d'adrénaline comparatives qui contenaient comme antioxydant de la vitamine E TPGS mais étaient dépourvues d'agent chélatant ;
- solutions C5 à C11 : 7 solutions d'adrénaline comparatives qui contenaient un ou plusieurs antioxydants différents de la vitamine E TPGS et qui étaient dépourvues d'agent chélatant ;
- solution C12 à C17: 5 solutions d'adrénaline comparatives qui contenaient un ou plusieurs antioxydants différents de la vitamine E TPGS, ainsi qu'un agent chélatant.

Les 19 solutions ont toutes été préparées de la manière suivante :
Tout d'abord, dans le cadre de cette préparation des 19 solutions, l'eau pour solution injectable qui a été utilisée a été soumise à un bullage de diazote pendant 12 heures afin supprimer toute trace de dioxygène résiduel (de telle sorte que sa teneur soit inférieure à 0,5 ppm).

Tous les constituants à l'exception de la substance active (à savoir l'adrénaline ou le tartrate d'adrénaline) ont été mélangés ensemble dans un flacon de 200 mL.

L'eau pour solution injectable a ensuite été ajoutée dans le flacon jusqu'à obtenir un volume de 180 mL.

La solution ainsi obtenue a été agitée avec un agitateur magnétique de manière à ce que tous les constituants se dissolvent.

Le pH a été ajusté à une valeur de 3,4 avec une solution soude (NaOH) ou d'acide chlorhydrique (HCl) à une concentration de 1 mol/L. Ces solutions avaient été soumises au préalable à un bullage avec du diazote.

Le volume de la solution a été complété à 200 mL avec de l'eau pour solution injectable. On a ainsi obtenu une 1^{ère} solution.

Ensuite, environ 1 mL de cette 1^{ère} solution a été utilisé pour dissoudre l'adrénaline ou, le cas échéant, le tartrate d'adrénaline dans un flacon.

La solution d'adrénaline ainsi obtenue a été transférée dans un flacon de 100 mL.

On a rincé plusieurs fois le flacon dans lequel a été dissoute l'adrénaline avec des faibles volumes (environ 1 mL) de la 1^{ère} solution, puis transféré cette solution de rinçage dans le flacon de 100 mL, et ce de manière à récupérer la totalité de l'adrénaline présente dans le flacon.

Enfin, à l'issue de ces rinçages, on a complété le volume à 100 mL avec la 1ère solution.

Les 19 solutions d'adrénaline ainsi obtenues étaient toutes limpides et incolores.

Dans les tableaux 1 à 3 ci-dessous, « Qsp » est l'abréviation de « quantité suffisante pour » signifiant que :
- la quantité de soude ou d'acide chlorhydrique à une concentration de 1 mol/L ajoutée était telle que le pH a été ajusté à environ 3,4 ;
- la quantité d'eau était telle que le volume de la solution a été complété à 100 mL.

Le tableau 1 ci-dessous détaille les quantités de chacun des constituants des solutions S1, S2, C1 à C4 pour un volume de 1 mL de chacune de ces solutions.

### Tableau 1]

**Tableau 1 détaillant les quantités de chacun des constituants des solutions S1, S2 et C1 à C4**

| **Constituants** | **S1** | **S2** | **C1** | **C2** | **C3** | **C4** |
|---|---|---|---|---|---|---|
| **Adrénaline (mg)** | 0 | | 0,6 | 0 | | |
| **Tartrate d'adrénaline (mg)** | 1,60 (équivalent à 0,88 d'adrénaline) | | 0 | 1,09 (équivalent à 0,6 d'adrénaline) | | |
| **NaCl (mg)** | 6 | | 8 | | 6 | |
| **Vitamine E TPGS (mg)** | 11 | 15 | 0 | | 7,5 | 3 |
| **EDTA disodique (mg)** | 1,5 | | 0 | | | |
| **NaOH ou HCl** | Qsp pH de 3,4 | | | | | |
| **Eau** | Qsp 1 mL | | | | | |

Le tableau 2 ci-dessous détaille les quantités de chacun des constituants des solutions C5 à C11 pour un volume de 1 mL de chacune de ces solutions.

### Tableau 2]

**Tableau 2 détaillant les quantités de chacun des constituants des solutions C5 à C11**

| **Constituants** | **C5** | **C6** | **C7** | **C8** | **C9** | **C10** | **C11** |
|---|---|---|---|---|---|---|---|
| **Adrénaline (mg)** | 0,88 | 0 | | | | | |
| **Tartrate d'adrénaline (mg)** | 0 | 1,09 (équivalent à 0,6 d'adrénaline) | | | | | |
| **NaCl (mg)** | 6 | | | | | | |
| **Méta-bisulfite de sodium (mg)** | 1,7 | 1 | 0,5 | 0 | | | |
| **L-cystéine (mg)** | 0 | | | 1 | 0 | 0 | 0 |
| **Acide citrique (mg)** | | | | 0 | 3 | 0 | 3 |
| **Monothioglycérol (mg)** | | | | 0 | 0 | 10 | 10 |
| **NaOH ou HCl** | Qsp pH de 3,4 | | | | | | |
| **Eau** | Qsp 1 mL | | | | | | |

Le tableau 3 ci-dessous détaille les quantités de chacun des constituants des solutions C12 à C17 pour un volume de 1 mL de chacune de ces solutions.

### Tableau 3]

**Tableau 3 détaillant les quantités de chacun des constituants des solutions C12 à C17**

| **Constituants** | **C12** | **C13** | **C14** | **C15** | **C16** | **C17** |
|---|---|---|---|---|---|---|
| **Tartrate d'adrénaline (mg)** | 1,09 (équivalent à 0,6 d'adrénaline) | 1,60 (équivalent à 0,88 d'adrénaline) | | 1,09 (équivalent à 0,6 d'adrénaline) | | |
| **NaCl (mg)** | 6 | | | | | |
| **Méta-bisulfite de sodium (mg)** | 1 | 1,5 | 2 | 0 | | |
| **L-cystéine (mg)** | 0 | | | 1 | | |
| **Acide citrique (mg)** | | | | 0 | 3 | |
| **Monothioglycérol (mg)** | | | 0 | 0 | 10 | |
| **EDTA disodique (mg)** | 1 | 1,5 | 1 | | | |
| **NaOH ou HCl** | Qsp pH de 3,4 | | | | | |
| **Eau** | Qsp 1 mL | | | | | |

La stabilité de ces 19 solutions d'adrénaline a été déterminée au cours du temps (à savoir jusqu'à 3 mois), et ce à différentes températures de stockage : 25°C, 40°C et 60°C.

Les tableaux 4 à 10 ci-dessous détaillent pour les solutions S1, S2 et C1 à 17, en fonction du temps et de la température de stockage :
- le pH de la solution ;
- l'apparence de la solution ;
- le pourcentage de perte de pureté de l'adrénaline dans la solution considérée.

Le pourcentage de perte de pureté de l'adrénaline dans la solution considérée a été déterminé par chromatographie liquide haute performance couplée à une détection ultra-violet selon la méthode détaillée dans la monographie du tartrate d'adrénaline n°01/2008:0254 issue de la 9^{ème} édition de la Pharmacopée Européenne.

### [Tableau 4]

**Tableau 4 détaillant les paramètres de stabilité des solutions S1 et S2**

| **Solution** | | | **Aspect** | **pH** | **% de perte pureté** |
|---|---|---|---|---|---|
| **S1** | **T0** | | Limpide et incolore | 3,37 | - |
| | **1 mois** | **25°C** | Limpide et incolore | 3,47 | 0 |
| | | **40°C** | Limpide et incolore | 3,47 | 0 |
| | | **60°C** | brun | 3,39 | 10,0 |
| | **2 mois** | **25°C** | Limpide et incolore | 3,45 | 0 |
| | | **40°C** | Limpide et incolore | 3,45 | 0,1 |
| | **3 mois** | **25°C** | Limpide et incolore | 3,43 | 0 |
| | | **40°C** | Limpide et incolore | 3,43 | 0,6 |
| **S2** | **T0** | | Limpide et incolore | 3,37 | - |
| | **1 mois** | **25°C** | Limpide et incolore | 3,47 | 0 |
| | | **40°C** | Limpide et incolore | 3,45 | 0 |
| | | **60°C** | brun | 3,40 | 6,5 |
| | **2 mois** | **25°C** | Limpide et incolore | 3,44 | 0 |
| | | **40°C** | Limpide et incolore | 3,42 | 0,1 |
| | **3 mois** | **25°C** | Limpide et incolore | 3,41 | 0 |
| | | **40°C** | Limpide et incolore | 3,42 | 0,3 |

Au vu du tableau 4, on relève que les solutions S1 et S2 selon l'invention demeurent stables au cours, et ce dans des conditions sévères en température. En effet, les solutions S1 et S2 commencent à se colorer à une température 60°C, à savoir une température élevée. On constate également les très faibles pourcentages de perte de pureté, à l'exception de la température très élevée de 60°C. En outre, on relève que le pH des solutions S1 et S2 ne varie quasiment pas, et ce même à 60°C.

Ces résultats expérimentaux témoignent de l'excellente stabilité des solutions d'adrénaline selon l'invention.

### Tableau 5]

**Tableau 5 détaillant les paramètres de stabilité des solutions C1 et C2**

| **Solution** | | | **Aspect** | **pH** | **% de perte pureté** |
|---|---|---|---|---|---|
| **C1** | **T0** | - | Limpide et incolore | 3,43 | - |
| | **2 semaines** | 60°C | marron foncé | 2,93 | 21,0 |
| | **1 mois** | 25°C | Ambré | 3,75 | 4,5 |
| | | 40°C | Ambré | 3,21 | 8,3 |
| **C2** | **T0** | | Limpide et incolore | 3,43 | - |
| | **2 semaines** | 60°C | marron | 3,11 | 26,6 |
| | **1 mois** | 25°C | Limpide et incolore | 3,42 | 1,8 |
| | | 40°C | orange | 3,28 | 13,9 |

Au vu du tableau 5, ces essais avec les solutions C1 et C2 témoignent que les solutions d'adrénaline dépourvues de tout antioxydant ne sont pas stables au cours du temps. En effet, les solutions C1 et C2 se sont colorées rapidement et le pourcentage de perte de pureté augmente avec le temps.

### [Tableau 6]

**Tableau 6 détaillant les paramètres de stabilité des solutions C3 et C4**

| **Solution** | | | Aspect | **pH** | **% de perte pureté** |
|---|---|---|---|---|---|
| **C3** | **T0** | - | Limpide et incolore | 3,43 | - |
| | **2 semaines** | 60°C | Légèrement jaune | 3,36 | 12,5 |
| | **1 mois** | 25°C | Limpide et incolore | 3,40 | 3,8 |
| | | 40°C | Limpide et incolore | 3,36 | 6,3 |
| | | 60°C | Marron | 3,30 | 25,9 |
| | **2 mois** | 25°C | Limpide et incolore | 3,42 | 3,9 |
| | | 40°C | Limpide et incolore | 3,39 | 8,9 |
| | **3 mois** | 25°C | Limpide et incolore | 3,41 | 4,5 |
| | | 40°C | Limpide et incolore | 3,41 | 11,6 |
| **C4** | **T0** | | Limpide et incolore | 3,39 | - |
| | **2 semaines** | 60°C | Marron foncé | 3,08 | 32,6 |
| | **1 mois** | 25°C | Légèrement jaune | 3,40 | 2,6 |
| | | 40°C | Ambré | 3,27 | 14,8 |

Au vu du tableau 6, ces essais avec les solutions C3 et C4 témoignent que les solutions d'adrénaline comprenant de la vitamine E TPGS mais qui sont dépourvues de tout agent chélatant présentent un pourcentage de perte de pureté qui augmente avec le temps et la température. Ces solutions C3 et C4 sont moins stables que les solutions S1 et S2 selon l'invention.

### [Tableau 7]

**Tableau 7 détaillant les paramètres de stabilité des solutions C5 à C7**

| **Solution** | | | **Aspect** | **pH** | **% de perte pureté** |
|---|---|---|---|---|---|
| **C5** | **T0** | - | Limpide et incolore | 3,43 | - |
| | **1 mois** | 25°C | Limpide et incolore | 3,00 | 2,8 |
| | | 40°C | Limpide et incolore | 2,63 | 8,4 |
| | | 60°C | Limpide et incolore | 2,73 | 25,3 |
| | **2 mois** | 25°C | Limpide et incolore | 2,90 | 4,4 |
| | | 40°C | Limpide et incolore | 2,78 | 13,2 |
| | **3 mois** | 25°C | Limpide et incolore | 2,82 | 5,2 |
| | | 40°C | Limpide et incolore | 2,61 | 15,3 |
| **C6** | **T0** | - | Limpide et incolore | 3,38 | - |
| | **2 semaines** | 60°C | Légèrement jaune | 2,27 | 13 |
| | **1 mois** | 25°C | Limpide et incolore | 3,34 | 1 |
| | | 40°C | Limpide et incolore | 2,76 | 8 |
| | | 60°C | Ambré | 2,29 | 20 |
| | **2 mois** | 25°C | Limpide et incolore | 3,19 | 2,5 |
| | | 40°C | Limpide et incolore | 2,60 | 10,7 |
| | **3 mois** | 25°C | Limpide et incolore | 3,08 | 4,9 |
| | | 40°C | Limpide et incolore | 2,54 | 13,8 |
| **C7** | **T0** | - | Limpide et incolore | 3,41 | - |
| | **2 semaines** | 60°C | Orange | 2,56 | 10,3 |
| | **1 mois** | 25°C | Limpide et incolore | 3,34 | 1,6 |
| | | 40°C | Limpide et incolore | 2,94 | 6,9 |
| | | 60°C | Ambré | 2,55 | 25,1 |
| | **2 mois** | 25°C | Limpide et incolore | 3,25 | 1,6 |
| | | 40°C | Limpide et incolore | 2,79 | 8,1 |
| | **3 mois** | 25°C | Limpide et incolore | 3,17 | 3,3 |
| | | 40°C | Limpide et incolore | 2,73 | 10,2 |

Au vu du tableau 7, ces essais avec les solutions C5 à C7 témoignent que les solutions d'adrénaline comprenant comme antioxydant du méta-bisulfite de sodium présentent un pourcentage de perte de pureté qui augmente avec le temps et la température. En outre, on relève que le pH varie au cours du temps et avec l'augmentation de la température. Ces solutions C5 à C7 ne sont pas stables au cours du temps.

### [Tableau 8]

**Tableau 8 détaillant les paramètres de stabilité des solutions C8 à C11**

| **Solution** | | | **Aspect** | **pH** | **% de perte pureté** |
|---|---|---|---|---|---|
| **C8** | **T0** | - | Limpide et incolore | 3,43 | - |
| | **2 semaines** | 60°C | Orange | 3,40 | 27,7 |
| | **1 mois** | 25°C | Légèrement jaune | 3,38 | 18,9 |
| | | 40°C | Jaune | 3,32 | 30,0 |
| | | 60°C | Orange | 3,39 | 41,0 |
| **C9** | **T0** | - | Limpide et incolore | 3,43 | - |
| | **2 semaines** | 60°C | Marron foncé | 3,31 | 24,3 |
| | **1 mois** | 25°C | Légèrement rose | 3,40 | 7,1 |
| | | 40°C | Orange | 3,36 | 11,1 |
| **C10** | **T0** | - | Limpide et incolore | 3,44 | - |
| | **2 semaines** | 60°C | Légèrement jaune | 3,21 | 63,4 |
| | **1 mois** | 25°C | Légèrement jaune | 3,28 | 62,5 |
| | | 40°C | Légèrement jaune | 3,19 | 65,4 |
| | | 60°C | Légèrement jaune | 3,23 | 81,7 |
| **C11** | **T0** | - | Limpide et incolore | 3,40 | - |
| | **2 semaines** | 60°C | Limpide et incolore | 3,31 | 52,8 |
| | **1 mois** | 25°C | Limpide et incolore | 3,33 | 52,5 |
| | | 40°C | Limpide et incolore | 3,33 | 58,0 |
| | | 60°C | Limpide et incolore | 3,34 | 76,9 |

Au vu du tableau 8, ces essais avec les solutions C8 à C11 témoignent que les solutions d'adrénaline comprenant comme antioxydant de la L-cystéine, l'acide citrique ou du monothioglycérol présentent un pourcentage de perte de pureté qui augmente avec le temps et la température. En outre, on relève que les solutions se colorent très rapidement. Ces solutions C8 à C11 ne sont pas stables au cours du temps.

### Tableau 9]

**Tableau 9 détaillant les paramètres de stabilité des solutions C12 à C14**

| **Solution** | | | Aspect | **pH** | **% de perte pureté** |
|---|---|---|---|---|---|
| **C12** | **T0** | - | Limpide et incolore | 3,40 | - |
| | **2 semaines** | 60°C | Limpide et incolore | 2,62 | 14,4 |
| | **1 mois** | 25°C | Limpide et incolore | 3,36 | 0,8 |
| | | 40°C | Limpide et incolore | 3,16 | 5,0 |
| | | 60°C | Limpide et incolore | 2,50 | 15,9 |
| | **2 mois** | 25°C | Limpide et incolore | 3,34 | 0,7 |
| | | 40°C | Limpide et incolore | 3,08 | 7,6 |
| | **3 mois** | 25°C | Limpide et incolore | 3,29 | 2,0 |
| | | 40°C | Limpide et incolore | 3,11 | 11,8 |
| **C13** | **T0** | - | Limpide et incolore | 3,42 | - |
| | **1 mois** | 25°C | Limpide et incolore | 3,47 | 0,9 |
| | | 40°C | Limpide et incolore | 3,30 | 5,5 |
| | | 60°C | Limpide et incolore | 3,03 | 25,7 |
| | **2 mois** | 25°C | Limpide et incolore | 3,42 | 2,0 |
| | | 40°C | Limpide et incolore | 3,23 | 10,8 |
| | **3 mois** | 25°C | Limpide et incolore | 3,42 | 2,6 |
| | | 40°C | Limpide et incolore | 2,99 | 14,0 |
| **C14** | **T0** | - | Limpide et incolore | 3,42 | - |
| | **1 mois** | 25°C | Limpide et incolore | 3,42 | 1,1 |
| | | 40°C | Limpide et incolore | 3,29 | 5,9 |
| | | 60°C | Marron | 3,55 | 29,3 |
| | **2 mois** | 25°C | Limpide et incolore | 3,40 | 2,2 |
| | | 40°C | Limpide et incolore | 3,26 | 10,5 |
| | **3 mois** | 25°C | Limpide et incolore | 3,36 | 2,8 |
| | | 40°C | Limpide et incolore | 3,32 | 13,6 |

Au vu du tableau 9, ces essais avec les solutions C12 à C14 témoignent que les solutions d'adrénaline comprenant comme antioxydant du méta-bisulfite de sodium en association avec un agent chélatant qui est l'EDTA disodique présentent un pourcentage de perte de pureté plus élevé que celui des solutions S1 et S2 au cours du temps et avec l'augmentation de la température. En outre, on relève que le pH varie au cours du temps et avec l'augmentation de la température pour les solutions C12 à C14 ; ce qui n'est pas le cas avec les solutions S1 et S2. Ces solutions C12 à C14 sont donc moins stables au cours du temps que les solutions S1 et S2.

### [Tableau 10]

**Tableau 10 détaillant les paramètres de stabilité des solutions C15 à C17**

| **Solution** | | | **Aspect** | **pH** | **% de perte pureté** |
|---|---|---|---|---|---|
| **C15** | **T0** | - | Limpide et incolore | 3,40 | - |
| | **2 semaines** | 60°C | Limpide et incolore | 3,37 | 40,0 |
| | **1 mois** | 25°C | Limpide et incolore | 3,39 | 8,2 |
| | | 40°C | Limpide et incolore | 3,37 | 9,9 |
| | | 60°C | Orange | 3,42 | 49,6 |
| **C16** | **T0** | - | Limpide et incolore | 3,35 | - |
| | **2 semaines** | 60°C | Limpide et incolore | 3,36 | 36,9 |
| | **1 mois** | 25°C | Limpide et incolore | 3,38 | 10,6 |
| | | 40°C | Limpide et incolore | 3,37 | 19,5 |
| | | 60°C | Marron | 3,41 | 48,2 |
| **C17** | **T0** | - | Limpide et incolore | 3,37 | - |
| | **2 semaines** | 60°C | Limpide et incolore | 3,26 | 54,5 |
| | **1 mois** | 25°C | Limpide et incolore | 3,32 | 51,9 |
| | | 40°C | Limpide et incolore | 3,29 | 55,8 |
| | | 60°C | Limpide et incolore | 3,29 | 79,5 |

Au vu du tableau 10, ces essais avec les solutions C15 à C17 témoignent que les solutions d'adrénaline comprenant comme antioxydant de la L-cystéine, de l'acide citrique et du monothioglycérol en association avec un agent chélatant qui est l'EDTA disodique présentent un pourcentage de perte de pureté beaucoup plus élevé que celui des solutions S1 et S2 après seulement un mois de stockage. Cette augmentation du pourcentage de perte de pureté s'accroit avec l'augmentation de la température. Ces solutions C15 à C17 sont donc beaucoup moins stables au cours du temps que les solutions S1 et S2.

## Revendications

1. Solution pharmaceutique d'adrénaline qui comprend au moins :
- de l'adrénaline ou un sel pharmaceutiquement acceptable de celle-ci ;
- du succinate de d-alpha-tocophéryl polyéthylène glycol (ci-après abrégé : « vitamine E TPGS ») ;
- un agent chélatant ;
- un solvant.

2. Solution pharmaceutique selon la revendication 1, **caractérisée en ce que** le sel pharmaceutiquement acceptable de l'adrénaline est choisi parmi le tartrate d'adrénaline et le chlorhydrate d'adrénaline.

3. Solution pharmaceutique selon la revendication 2, **caractérisée en ce que** le sel pharmaceutiquement acceptable de l'adrénaline est le tartrate d'adrénaline.

4. Solution pharmaceutique selon l'une quelconque des revendications 1 à 3, **caractérisée en ce que** l'agent chélatant est choisi parmi l'éthylènediaminetétraacétate disodique (ci-après abrégé « EDTA disodique »), l'acide éthylènediaminetétraacétique et l'éthylènediaminetétraacétate de calcium disodique.

5. Solution pharmaceutique selon la revendication 4, **caractérisée en ce que** l'agent chélatant est l'EDTA disodique.

6. Solution pharmaceutique selon l'une quelconque des revendications 1 à 5, **caractérisée en ce qu'**elle comprend en outre au moins un agent tampon de pH qui est choisi parmi l'acide chlorhydrique et la soude.

7. Solution pharmaceutique selon l'une quelconque des revendications 1 à 6, **caractérisée en ce que** le pH de la solution pharmaceutique est compris entre 2,2 et 5, de préférence entre 3 et 3,8.

8. Solution pharmaceutique selon l'une quelconque des revendications 1 à 7, **caractérisée en ce qu'**elle comprend en outre au moins un modificateur de tonicité qui est du chlorure de sodium.

9. Solution pharmaceutique selon l'une quelconque des revendications 1 à 8, **caractérisée en ce que** la concentration en adrénaline dans ladite solution pharmaceutique est comprise entre 0,1 mg/mL et 1 mg/mL.

10. Solution pharmaceutique selon l'une quelconque des revendications 1 à 9, **caractérisée en ce qu'**elle comprend en mg pour 1 mL de solution :
- entre 0,1 mg et 1 mg, plus préférentiellement entre 0,48 et 0,8 mg, d'adrénaline ou d'un sel pharmaceutiquement acceptable de celle-ci ;
- entre 0,1 mg et 20 mg, de préférence entre 2,5 mg et 15 mg, de vitamine E TPGS ;
- entre 0,1 mg et 2 mg, de préférence entre 1 mg et 1,5 mg, d'agent chélatant ;
- Q.S.P. 1 mL de solvant, de préférence d'eau pour préparation injectable.

11. Procédé de préparation de la solution pharmaceutique selon l'une quelconque des revendications 1 à 10, **caractérisé en ce qu'**il comprend au moins les étapes suivantes :
a) on prépare sous agitation un mélange comprenant tous les constituants de ladite solution à l'exception de l'adrénaline ou du sel pharmaceutiquement acceptable d'adrénaline de manière à les dissoudre ;
b) on dissout dans un solvant l'adrénaline ou un sel pharmaceutiquement acceptable de celle-ci ;
c) on ajoute au mélange de l'étape a), optionnellement sous agitation, l'adrénaline dissoute ou le sel pharmaceutiquement acceptable de celle-ci dissout.

12. Solution pharmaceutique selon l'une quelconque des revendications 1 à 10 pour son utilisation dans le traitement des chocs anaphylactiques, des arrêts cardiaques, de l'asthme et des détresses cardio-circulatoires.

13. Solution pharmaceutique selon la revendication 12, **caractérisée en ce que** ladite solution est sous une forme adaptée à l'administration par voie parentérale, de préférence par voie intramusculaire.

14. Kit d'injection, de préférence kit d'injection par voie intramusculaire, comportant :
- un dispositif d'injection ;
- la solution pharmaceutique selon l'une quelconque des revendications 1 à 10.

15. Kit d'injection selon la revendication 14, **caractérisé en ce que** le dispositif d'injection est un dispositif d'injection sans aiguille à cartouche pyrotechnique.

## Patentansprüche

1. Pharmazeutische Adrenalin-Lösung, die mindestens umfasst:
- Adrenalin oder ein pharmazeutisch akzeptables Salz davon;
- D-alpha-Tocopherylpolyethylenglykolsuccinat (nachfolgend abgekürzt: "Vitamin E TPGS");
- ein chelatbildendes Mittel;
- ein Lösungsmittel.

2. Pharmazeutische Lösung nach Anspruch 1, **dadurch gekennzeichnet, dass** das pharmazeutisch akzeptable Salz des Adrenalins ausgewählt ist aus Adrenalintartrat und Adrenalinhydrochlorid.

3. Pharmazeutische Lösung nach Anspruch 2, **dadurch gekennzeichnet, dass** es sich bei dem pharmazeutisch akzeptablen Salz des Adrenalins um Adrenalintartrat handelt.

4. Pharmazeutische Lösung nach einem der Ansprüche 1 bis 3, **dadurch gekennzeichnet, dass** das chelatbildende Mittel ausgewählt ist aus Dinatrium-Ethylendiamintetraacetat (nachfolgend abgekürzt "Dinatrium-EDTA"), Ethylendiamintetraessigsäure, und Calcium-Dinatrium-Ethylendiamintetraacetat.

5. Pharmazeutische Lösung nach Anspruch 4, **dadurch gekennzeichnet, dass** es sich bei dem chelatbildenden Mittel um Dinatrium-EDTA handelt.

6. Pharmazeutische Lösung nach einem der Ansprüche 1 bis 5, **dadurch gekennzeichnet, dass** sie weiter mindestens ein pH-Puffermittel umfasst, das aus Salzsäure und Soda ausgewählt ist.

7. Pharmazeutische Lösung nach einem der Ansprüche 1 bis 6, **dadurch gekennzeichnet, dass** der pH-Wert der pharmazeutischen Lösung im Bereich zwischen 2,2 und 5, vorzugsweise zwischen 3 und 3,8 liegt.

8. Pharmazeutische Lösung nach einem der Ansprüche 1 bis 7, **dadurch gekennzeichnet, dass** sie weiter mindestens einen Tonizitätsmodifikator umfasst, bei dem es sich um Natriumchlorid handelt.

9. Pharmazeutische Lösung nach einem der Ansprüche 1 bis 8, **dadurch gekennzeichnet, dass** die Konzentration von Adrenalin in der pharmazeutischen Lösung im Bereich zwischen 0,1 mg/mL und 1 mg/mL liegt.

10. Pharmazeutische Lösung nach einem der Ansprüche 1 bis 9,
**dadurch gekennzeichnet, dass** sie in mg pro 1 mL Lösung umfasst:
- zwischen 0,1 mg und 1 mg, bevorzugter zwischen 0,48 und 0,8 mg Adrenalin oder eines pharmazeutisch akzeptablen Salzes davon;
- zwischen 0,1 mg und 20 mg, vorzugsweise zwischen 2,5 mg und 15 mg Vitamin E TPGS;
- zwischen 0,1 mg und 2 mg, vorzugsweise zwischen 1 mg und 1,5 mg chelatbildendes Mittel;
- Q.S.P. 1 mL Lösungsmittel, vorzugsweise Wasser für Injektionszwecke.

11. Verfahren zur Herstellung der pharmazeutischen Lösung nach einem der Ansprüche 1 bis 10, **dadurch gekennzeichnet, dass** es mindestens die folgenden Schritte umfasst:
a) Herstellen einer Mischung, die alle Bestandteile der Lösung mit Ausnahme des Adrenalins oder des pharmazeutisch akzeptablen Salzes von Adrenalin umfasst, unter Rühren, um sie zu lösen;
b) Lösen des Adrenalins oder eines pharmazeutisch akzeptablen Salzes davon in einem Lösungsmittel;
c) Zugeben des gelösten Adrenalins oder des gelösten pharmazeutisch akzeptablen Salzes davon, gegebenenfalls unter Rühren, zu der Mischung aus Schritt a).

12. Pharmazeutische Lösung nach einem der Ansprüche 1 bis 10 zu deren Verwendung bei der Behandlung von anaphylaktischen Schocks, Herzstillständen, Asthma und Herz-Kreislauf-Notfällen.

13. Pharmazeutische Lösung nach Anspruch 12, **dadurch gekennzeichnet, dass** die Lösung in einer Form vorliegt, die für die parenterale, vorzugsweise intramuskuläre Verabreichung geeignet ist.

14. Injektionsset, vorzugsweise Set für die intramuskuläre Injektion, umfassend:
- eine Injektionsvorrichtung;
- die pharmazeutische Lösung nach einem der Ansprüche 1 bis 10.

15. Injektionsset nach Anspruch 14, **dadurch gekennzeichnet, dass** es sich bei der Injektionsvorrichtung um eine nadellose Injektionsvorrichtung mit pyrotechnischer Patrone handelt.

## Claims

1. An adrenaline pharmaceutical solution comprising at least:
- adrenaline or a pharmaceutically-acceptable salt thereof;
- D-alpha-tocopheryl polyethylene glycol succinate (hereinafter, abbreviated as « vitamin E TPGS »);
- a chelating agent;
- a solvent.

2. The pharmaceutical solution according to claim 1, **characterized in that** the pharmaceutically-acceptable salt of adrenaline is selected from adrenaline tartrate and adrenaline hydrochloride.

3. The pharmaceutical solution according to claim 2, **characterized in that** the pharmaceutically-acceptable salt of adrenaline is adrenaline tartrate.

4. The pharmaceutical solution according to any one of claims 1 to 3, **characterized in that** the chelating agent is selected from disodium ethylenediaminetetraacetate (hereinafter, abbreviated as « disodium EDTA »), ethylenediaminetetraacetic acid and disodium calcium ethylenediaminetetraacetate.

5. The pharmaceutical solution according to claim 4, **characterized in that** the chelating agent is disodium EDTA.

6. The pharmaceutical solution according to any one of claims 1 to 5, **characterized in that** it further comprises at least one pH buffering agent which is selected from hydrochloric acid and soda.

7. The pharmaceutical solution according to any one of claims 1 to 6, **characterized in that** the pH of the pharmaceutical solution is comprised between 2.2 and 5, preferably between 3 and 3.8.

8. The pharmaceutical solution according to any one of claims 1 to 7, **characterized in that** it further comprises at least one tonicity modifier which is sodium chloride.

9. The pharmaceutical solution according to any one of claims 1 to 8, **characterized in that** the adrenaline concentration in said pharmaceutical solution is comprised between 0.1 mg/mL and 1 mg/ml.

10. The pharmaceutical solution according to any one of claims 1 to 9, **characterized in that** it comprises in mg for 1 mL of the solution:
- between 0.1 mg and 1 mg, more preferably between 0.48 and 0.8 mg, of adrenaline or of a pharmaceutically-acceptable salt thereof;
- between 0.1 mg and 20 mg, preferably between 2.5 mg and 15 mg, of vitamin E TPGS;
- between 0.1 mg and 2 mg, preferably between 1 mg and 1.5 mg, of a chelating agent;
- Q.S. 1 mL of solvent, preferably water for injection preparation.

11. A process for preparing the pharmaceutical solution according to any one of claims 1 to 10, **characterized in that** it comprises at least the following steps of:
a) preparing, under stirring, a mixture comprising all of the constituents of said solution except adrenaline or the pharmaceutically-acceptable salt of adrenaline so as to dissolve them;
b) dissolving adrenaline or a pharmaceutically-acceptable salt thereof in a solvent;
c) adding to the mixture of step a), optionally under stirring, the dissolved adrenaline or the dissolved pharmaceutically-acceptable salt thereof.

12. A pharmaceutical solution according to any one of claims 1 to 10 for its use in the treatment of anaphylactic shocks, cardiac arrests, asthma and cardiocirculatory distresses.

13. The pharmaceutical solution according to claim 12, **characterized in that** said solution is in a form suitable for the administration by parenteral route, preferably by intramuscular route.

14. An injection kit, preferably a kit for injection by intramuscular route, including:
- an injection device;
- the pharmaceutical solution according to any one of claims 1 to 10.

15. The injection kit according to claim 14, **characterized in that** the injection device is a needleless injection device with a pyrotechnic cartridge.
